# EUROPEAN PATENT APPLICATION

(11) **EP 4 487 900 A1**
(43) Date of publication of application: **08.01.2025**
(21) Application number: 23763421.7
(22) Date of filing: 28.02.2023
(51) Int. Cl.: A61N 1/32, B26B 19/48

(54) **BEAUTY DEVICE AND ELECTRIC RAZOR**

(30) Priority: 03.03.2022 JP 2022032763
(71) Applicant: Panasonic Intellectual Property Management Co., Ltd., Kadoma-shi, Osaka 571-0057 (JP)
(72) Inventor: MATSUDA Misaki, Kadoma-shi, Osaka 571-0057 (JP); SHIMIZU Hiroaki, Kadoma-shi, Osaka 571-0057 (JP); NAKAGAWA Takehiro, Kadoma-shi, Osaka 571-0057 (JP)
(74) Representative: Novagraaf International SA
(86) International application number: PCT/JP2023/007200
(87) International publication number: WO 2023/167163

(57) **Abstract**

The present disclosure provides a beauty device capable of applying electricity to skin of a user with an appropriate current value. Beauty device (1) according to the present disclosure is a beauty device that applies electricity to skin of a user. Beauty device (1) includes electrode unit (30) that is brought into contact with skin of a user to apply the electricity to the skin of the user. Beauty device (1) includes a current value switching unit that switches a current value for applying electricity to the skin of the user from electrode unit (30). Beauty device (1) includes a current value controller that controls the current value to be applied from electrode unit (30) based on the current value switched by current value switching unit (12). The current value controller controls the current value such that an application level of the current value to be applied from electrode unit (30) becomes a value in a range of 100 µA to 700 µA.

## Description

### TECHNICAL FIELD

The present disclosure relates to a beauty device and an electric razor.

### BACKGROUND ART

A beauty device in which an electrode unit is brought into contact with skin of a user and electricity is applied from the electrode unit to the skin of the user has been proposed. PTL 1 discloses a beauty device in which an electrode formed in a convex shape is brought into contact with skin of a user to apply electricity to the skin of the user. The beauty device disclosed in PTL 1 performs treatment on details of the skin of the user by applying electricity at three levels of weak, medium, and strong in accordance with user's preference.

### Citation List

### Patent Literature

PTL 1: Unexamined Japanese Patent Publication No. 2016-32516

### SUMMARY OF THE INVENTION

### Technical problem

The beauty device disclosed in PTL 1 can change intensity of the electricity applied to the skin via the electrode unit in three electrode application modes. However, since a resistance value in the human body varies depending on the user, how the user feels in a case where a voltage of the same intensity is applied varies depending on the user. That is, an effect of the beauty device increases as a current value of the electricity to be applied increases, but stimulation by the application may cause discomfort depending on a person. Thus, a beauty device having a good balance between the stimulation and the effect is required for the user.

The present disclosure has been made in view of such a problem of the background art. An object of the present disclosure is to provide a beauty device capable of applying electricity to skin of a user with an appropriate current value.

### Solution to problem

A beauty device according to an aspect of the present disclosure is a beauty device that applies an electricity to skin of a user. The device includes an electrode unit that is brought into contact with the skin of the user to apply the electricity to the skin of the user, a current value switching unit that switches a current value to be applied to the skin of the user from the electrode unit, and a current value controller that controls the current value to be applied from the electrode unit based on the current value switched by the current value switching unit. The current value controller controls the current value such that an application level of the current value to be applied from the electrode unit is a value in a range of 100 µA to 700 µA.

An electric razor according to another aspect of the present disclosure includes the beauty device described above.

### Advantageous effect of invention

According to the present disclosure, it is possible to provide the beauty device capable of applying electricity to the skin of the user with an appropriate current value.

### BRIEF DESCRIPTION OF THE DRAWINGS

Fig. 1A is a perspective view illustrating a configuration of a beauty device according to a first exemplary embodiment.
Fig. 1B is a front view illustrating a configuration of the beauty device according to the first exemplary embodiment.
Fig. 2 is a block diagram illustrating the configuration of the beauty device according to the first exemplary embodiment.
Fig. 3 is a block diagram illustrating a functional configuration of a control board of the beauty device according to the first exemplary embodiment.
Fig. 4 is a diagram for explaining a resistor of the beauty device according to the first exemplary embodiment.
Fig. 5 is a graph for explaining a current value of the beauty device according to the first exemplary embodiment.
Fig. 6A is a graph for explaining a relationship between the current value and a skin care effect by application in the beauty device according to the first exemplary embodiment.
Fig. 6B is a graph for explaining a relationship between the current value and stimulation of the beauty device according to the first exemplary embodiment.
Fig. 7 is a block diagram illustrating a configuration of a beauty device according to a second exemplary embodiment.
Fig. 8A is a diagram for explaining a resistor of the beauty device according to the second exemplary embodiment.
Fig. 8B is a diagram for explaining the resistor of the beauty device according to the second exemplary embodiment.
Fig. 9 is a front view illustrating a configuration of a beauty device according to another exemplary embodiment.

### DESCRIPTION OF EMBODIMENT

Hereinafter, exemplary embodiments will be explained in detail with reference to some drawings. Note that, descriptions more in detail than necessary are sometimes omitted. For example, detailed descriptions of already well-known matters or redundant descriptions of substantially the same configuration may be omitted. The accompanying drawings and the following description are provided for those skilled in the art to fully understand the present disclosure, and are not intended to limit the subject matter described in the claims.

### (Schematic configuration of beauty device)

A beauty device according to the present exemplary embodiment is a device for performing skin care of a user by being held by the user, bringing a part of the beauty device into contact with the skin of the user to apply electricity to the skin of the user. Hereinafter, the beauty device will be described with reference to some specific exemplary embodiments.

### (First exemplary embodiment)

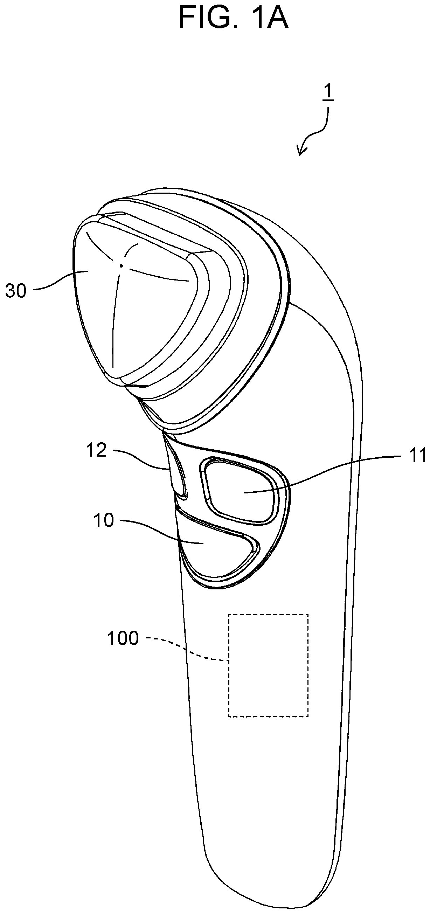

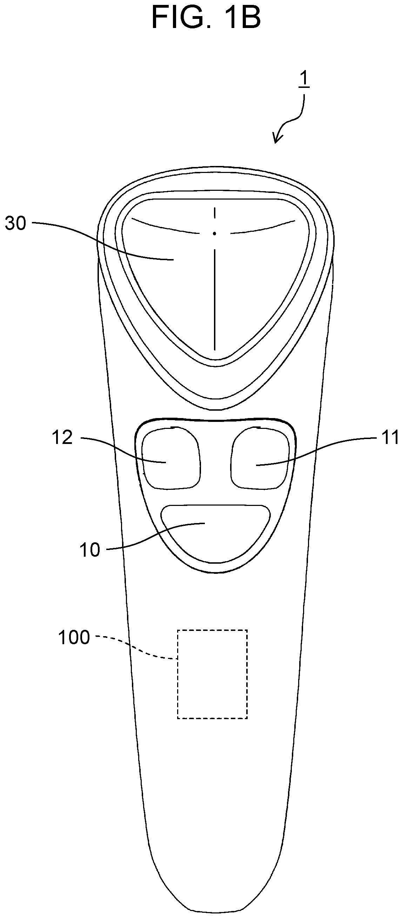

Fig. 1A is a perspective view illustrating a configuration of beauty device 1 according to a first exemplary embodiment. In addition, Fig. 1B is a front view illustrating the configuration of beauty device 1 according to the first exemplary embodiment.

As illustrated in Figs. 1A and 1B, beauty device 1 includes mode switching unit 10, temperature switching unit 11, and current value switching unit 12. In the first exemplary embodiment, mode switching unit 10, temperature switching unit 11, and current value switching unit 12 are present on an outer shell of beauty device 1, and each include various switches. In addition, beauty device 1 includes electrode unit 30 that is brought into contact with the skin of the user to apply electricity to the skin of the user. In addition, beauty device 1 includes control board 100 therein.
electrode unit 30 can be made of metal that is a conductive material. In this case, it is preferable to form the electrode unit by using a metal having a low influence on the human body. In the first exemplary embodiment, electrode unit 30 is made of titanium, which can suppress the occurrence of a problem of metal allergy.

Mode switching unit 10 includes a switch that can switch an operation mode of beauty device 1. In addition, in a case where a predetermined mode is selected, temperature switching unit 11 and current value switching unit 12 are switches capable of setting a temperature and a current value in accordance with user's preference.

A cleansing mode, a moisture keeping mode, and a vibration mode are provided, as modes, in beauty device 1 according to the first exemplary embodiment. These modes are switched by pressing the switch that is mode switching unit 10.

The cleansing mode is a mode in which cleansing is performed by ion derivation by covering electrode unit 30 with a treatment member (not illustrated) such as a cotton sheet impregnated with a serum and bringing electrode unit 30 covered with the treatment member into contact with the skin.

The moisture keeping mode is a mode for causing the serum to permeate the skin by applying a predetermined voltage to electrode unit 30 and generating a potential difference between the skin and electrode unit 30.

The vibration mode is a mode for treating the skin by applying the serum to the skin and then vibrating electrode unit 30.

Temperature switching unit 11 is a switch for controlling the temperature in each mode switched by mode switching unit 10. In each mode, the user presses the switch that is temperature switching unit 11, and thus, a temperature of electrode unit 30 is switched between low, high, and off.

Current value switching unit 12 is a switch for switching a current value to be applied to the skin from electrode unit 30 in each mode switched by mode switching unit 10. In the present exemplary embodiment, an application level of the current value to be applied to the skin from electrode unit 30 is 100 µA (microamperes) to 700 µA. That is, current value switching unit 12 is a switch that allows the user to freely set the application level of the current value flowing from electrode unit 30 to be in a range of 100 µA to 700 µA.

In the first exemplary embodiment, the switch used in current value switching unit 12 may include a button switch. In this case, in a state where the user presses the switch, the current value increases from 100 µA to 700 µA, and the current value increases again from 100 µA after reaching 700 µA. In addition, the switch used in current value switching unit 12 may be a rotary switch that rotates a knob to adjust the current value to a predetermined value. In this case, the user can switch the current value to be applied to the skin from electrode unit 30 by adjusting a value indicated by the rotary switch to a desired current value. In addition, the switch used in current value switching unit 12 may be a slide switch, and may be configured to slide the switch to a desired current value.

In each mode, the user presses or adjusts the switch that is current value switching unit 12, and thus, the current value to be applied to the skin from electrode unit 30 is switched to a predetermined value. Details of the switching of the current value in the first exemplary embodiment will be described later.

### (Configuration and function of beauty device 1)

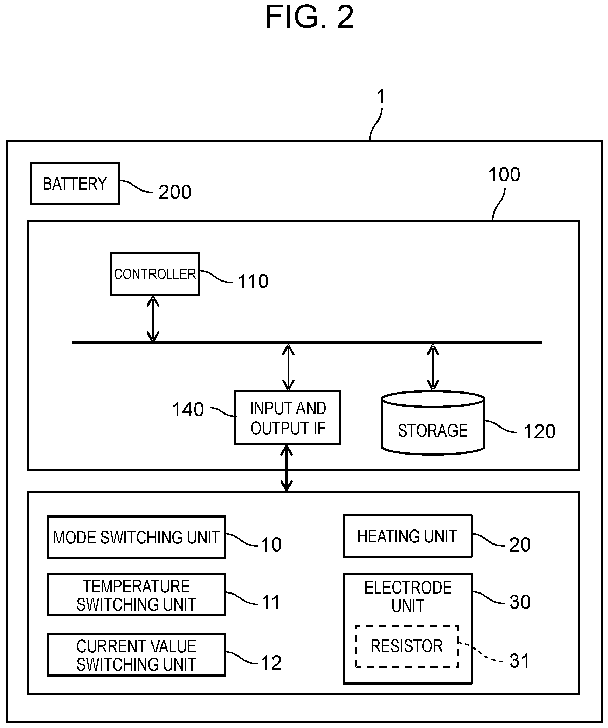

Fig. 2 is a block diagram illustrating the configuration of beauty device 1 according to the first exemplary embodiment. As described above, beauty device 1 includes, as the switches, mode switching unit 10, temperature switching unit 11, and current value switching unit 12. In addition, beauty device 1 includes electrode unit 30. Further, as illustrated in Fig. 2, beauty device 1 further includes control board 100, battery 200, and heating unit 20. In the present specification, the abbreviation "IF" means an interface. In the present specification, the abbreviation "DB" means a database. In the present specification, the abbreviation "CPU" means a central processing unit. In the present specification, the abbreviation "ROM" means a read-only memory.

Control board 100 includes controller 110, storage 120, and input and output IF 140. Control board 100 may be a board including a general-purpose microcomputer including a CPU (that is, an example of controller 110), a memory (that is, an example of storage 120), and input and output IF 140. In this case, a computer program for causing the microcomputer to function as beauty device 1 may be installed. The computer program is executed, and thus, the microcomputer functions as a plurality of information processing circuits included in beauty device 1.

In the present exemplary embodiment, although an example in which the plurality of information processing circuits included in beauty device 1 are realized by software is illustrated, the information processing circuit can also be realized by preparing dedicated hardware for executing each information processing to be described below. In addition, the plurality of information processing circuits may be realized by individual hardware.

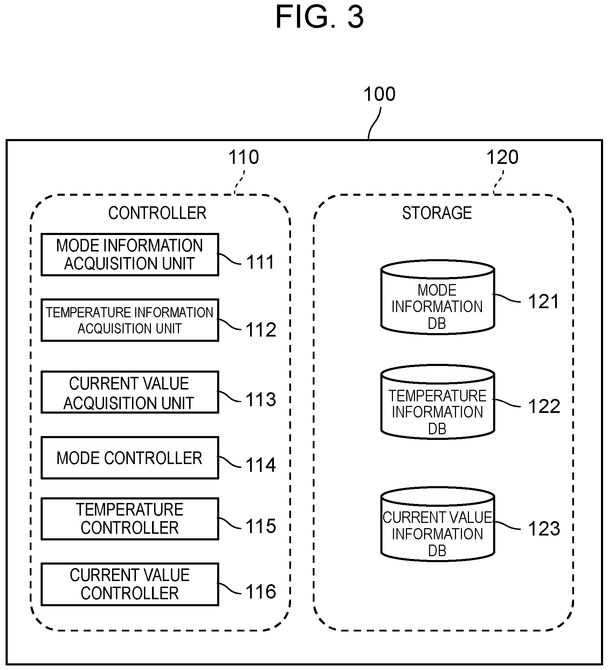

Controller 110 operates based on programs (not illustrated) stored in storage 120, and executes functions of beauty device 1. The programs are not limited to be stored in storage 120, and may be stored in, for example, a ROM (not illustrated) within control board 100. Fig. 3 is a block diagram illustrating a functional configuration of control board 100 of beauty device 1 according to the first exemplary embodiment. As illustrated in Fig. 3, controller 110 includes, as functions, mode information acquisition unit 111, temperature information acquisition unit 112, current value acquisition unit 113, mode controller 114, temperature controller 115, and current value controller 116.

Storage 120 acquires information regarding the mode, the temperature, and the current value designated by pressing the corresponding switch via input and output IF 140, and stores, as mode information DB 121, temperature information DB 122, and current value information DB 123, the mode, the temperature, and the current value, respectively, in storage 120 as illustrated in Fig. 3. In addition, as described above, storage 120 may store the program for each function executed in controller 110. At least one of the information regarding the pressing of each switch and the program stored in storage 120 may be a region physically or logically provided separately in one storage device. Alternatively, storages 120 of pieces of data may be provided in a plurality of physically different storage devices.

Input and output IF 140 is an interface for transmitting and receiving data and a control signal between control board 100 and each switch of beauty device 1. In addition, input and output IF 140 may function as a component (that is, the interface) for the user to exchange data with beauty device 1. For example, the user can update the function of beauty device 1 via input and output IF 140.

Heating unit 20 switches the temperature of electrode unit 30 in accordance with the temperature set by temperature switching unit 11 described above. Specifically, heating unit 20 includes a heater (not illustrated), a heat transfer plate (not illustrated), and a temperature detection element (not illustrated). In the first exemplary embodiment, a heat transfer plate having substantially the same shape as an inner surface shape of electrode unit 30 is disposed inside electrode unit 30 via a heat transfer material, and a temperature detection element that detects the temperature is sandwiched between the inside of electrode unit 30 and the heat transfer plate.

In addition, a surface of the heat transfer plate opposite to electrode unit 30 is a flat surface, and the heat transfer plate is formed such that the heater can be brought into contact with the flat surface without a gap. Further, the heater is configured to be biased forward (that is, biased toward electrode unit 30) by a reaction force of a heater holding spring (not illustrated) housed in a heater holding spring housing portion (not illustrated) formed in the electrode holding plate.

The heater holding spring is, for example, a coil spring, and is configured to bias the heater forward (that is, to bias toward electrode unit 30) in a state of being inserted through a protrusion (not illustrated) formed in the heater holding spring housing portion.

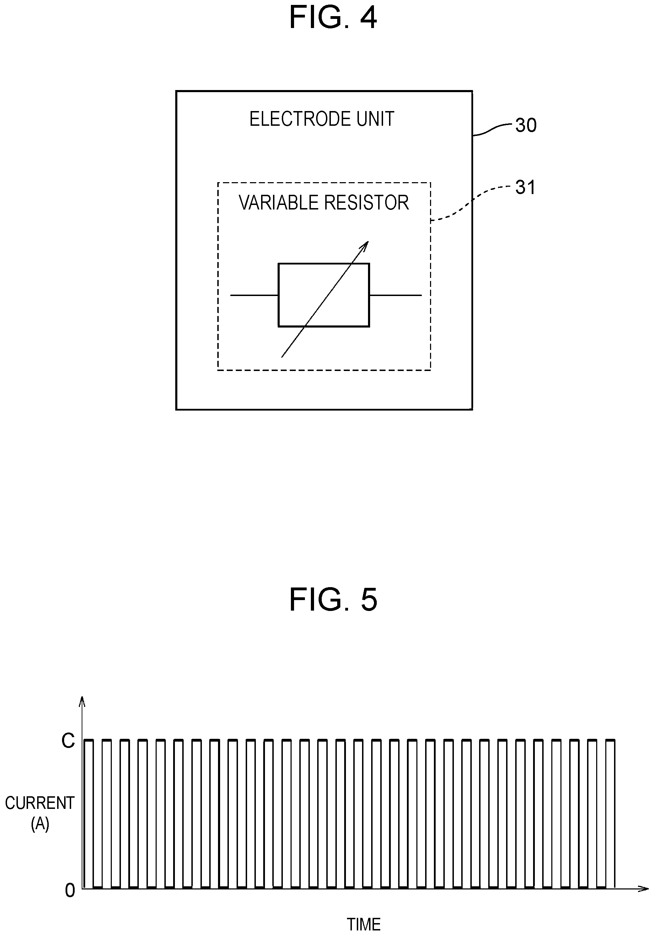

As described above, electrode unit 30 has a function of being brought into contact with the skin of the user to apply electricity to the skin. Fig. 4 is a diagram for explaining resistor 31 of beauty device 1 according to the first exemplary embodiment. In the first exemplary embodiment, electrode unit 30 includes a variable resistor as resistor 31 as illustrated in Fig. 4. In the first exemplary embodiment, controller 110 changes a resistance value of the variable resistor with respect to the current value set by pressing current value switching unit 12, and thus, electrode unit 30 switches the value of the current applied from electrode unit 30 to the skin of the user.

Fig. 5 illustrates an example of the current applied from electrode unit 30 to the skin of the user. In the first exemplary embodiment, electrode unit 30 applies, as a pulse current or a sinusoidal current of a predetermined cycle, current C that is a predetermined current value to the skin of the user. Here, in the present exemplary embodiment, current C that is the predetermined current value is a current value that can be set in a range of 100 µA to 700 µA.

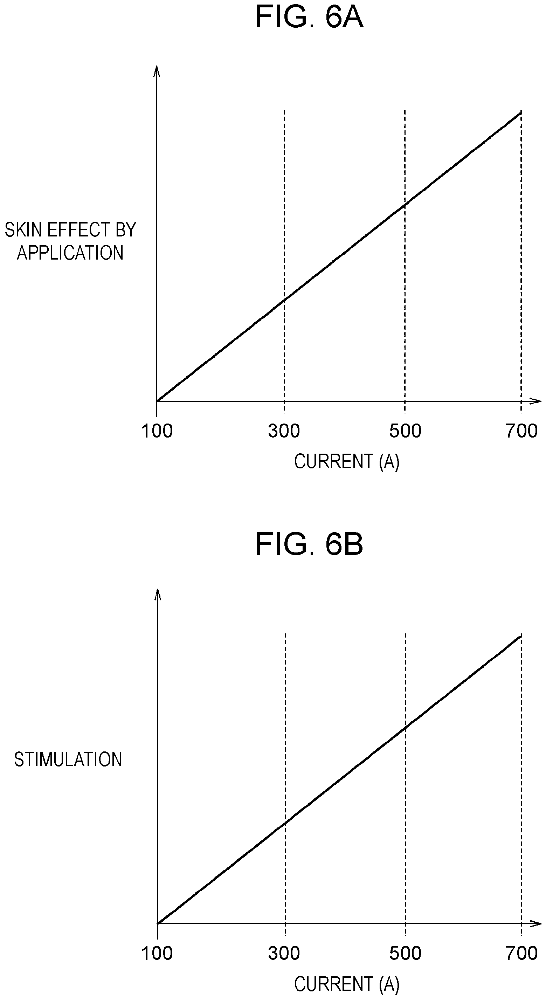

Fig. 6A is a diagram for explaining a relationship between the current value set for electrode unit 30 and a skin care effect in the application. In addition, Fig. 6B is a diagram for explaining a relationship between the current value set for electrode unit 30 and stimulation to the skin. As illustrated in Fig. 6A, the larger the value of the current set for electrode unit 30 and applied to the skin of the user, the higher the skin care effect in the application. Similarly, as illustrated in Fig. 6B, the larger the value of the current set for electrode unit 30 and applied to the skin of the user, the higher the stimulation in the application.

That is, in the example illustrated in Figs. 6A and 6B, in a case where the current value to be applied to the skin is from 100 µA to 300 µA, the skin care effect is low, but stimulation is suppressed. That is, in a case where the current value to be applied to the skin is from 100 µA to 300 µA, the current value is appropriate for the user suitable for weak stimulation by the application.

In addition, in the example illustrated in Figs. 6A and 6B, in a case where the current value to be applied to the skin is from 300 µA to 500 µA, balance between the skin care effect and the stimulation is good. That is, in a case where the current value to be applied to the skin is from 300 µA to 500 µA, the current value is appropriate for the user who wants to obtain stimulation by the application and the skin care effect in a well-balanced manner.

Further, in the example illustrated in Figs. 6A and 6B, in a case where the current value to be applied to the skin is from 500 µA to 700 µA, the skin care effect is high. That is, in a case where the current value to be applied to the skin is from 500 µA to 700 µA, although discomfort caused by the application may be increased depending on the user, the current value is appropriate for the user who wants to obtain a high skin care effect by the application.

Battery 200 supplies predetermined power to control board 100, heating unit 20, and electrode unit 30. That is, control board 100, heating unit 20, and electrode unit 30 perform various operations based on power supplied from battery 200. In addition, battery 200 may be configured to supply power to at least one of mode switching unit 10, temperature switching unit 11, and current value switching unit 12.

### (Functional configuration of beauty device 1)

Next, a functional configuration of beauty device 1 will be described. As described above, controller 110 includes, as the functions, mode information acquisition unit 111, temperature information acquisition unit 112, current value acquisition unit 113, mode controller 114, temperature controller 115, and current value controller 116.

Mode information acquisition unit 111 acquires mode information set by the pressing of mode switching unit 10, and stores the mode information in mode information DB 121 of storage 120.

Temperature information acquisition unit 112 acquires temperature information set by the pressing of temperature switching unit 11, and stores the temperature information in temperature information DB 122 of storage 120.

Current value acquisition unit 113 acquires a current value set by the pressing of current value switching unit 12, and stores the current value in current value information DB 123 of storage 120.

Based on the mode information stored in mode information DB 121, mode controller 114 switches beauty device 1 to one of the cleansing mode, the moisture keeping mode, and the vibration mode.

Temperature controller 115 controls heating unit 20 based on the temperature information stored in temperature information DB 122 to switch the temperature of electrode unit 30 of beauty device 1.

Current value controller 116 switches the application level of the current value to be applied to the skin from electrode unit 30 based on the current value stored in the current value information DB 123. In the first exemplary embodiment, current value controller 116 can set a current in a range of 100 µA to 700 µA as an application level of a current value to be applied from electrode unit 30 to the skin by controlling the variable resistor of electrode unit 30. That is, current value controller 116 controls the current value such that the application level of the current value to be applied from electrode unit 30 becomes a value in the range of 100 µA to 700 µA.

In addition, current value information DB 123 stores a current value when beauty device 1 is previously used by the user. In addition, when beauty device 1 is started, current value controller 116 sets the current value of electrode unit 30 based on current value information that is the previously used current value and stored in current value information DB 123. Thus, the user does not need to set an appropriate current value for each treatment, and can operate beauty device 1 with an optimal current value.

As described above, beauty device 1 according to the first exemplary embodiment is a beauty device that applies electricity to the skin of the user. Beauty device 1 includes electrode unit 30 that is brought into contact with the skin of the user to apply electricity to the skin of the user. In addition, beauty device 1 includes current value switching unit 12 that switches a current value to be applied to the skin of the user from electrode unit 30. In addition, beauty device 1 also includes controller 110 that controls the current value to be applied from electrode unit 30 based on the current value switched by current value switching unit 12. Controller 110 controls the current value such that the application level of the current value to be applied from electrode unit 30 becomes a value in the range of 100 µA to 700 µA.

Thus, beauty device 1 can apply the electricity to the skin of the user with an appropriate current value in accordance with the user by controlling the current value such that the application level of the current value to be applied from electrode unit 30 is in the range of 100 µA to 700 µA.

In addition, current value switching unit 12 may be present on the outer shell of beauty device 1, and thus, the current value is changeable by the user. Thus, in beauty device 1, the current value can be changed in accordance with user's preference, and electricity can be applied to the skin of the user with an appropriate current value in accordance with the user.

In addition, in the first exemplary embodiment, storage 120 may store the previously used current value. Thus, the user does not need to set an appropriate current value for beauty device 1 for each treatment, and beauty device 1 can be operated with an optimal current value.

In addition, current value switching unit 12 may be a switch that allows the user to freely set the application level of the current value flowing from electrode unit 30 to be in the range of 100 µA to 700 µA. Thus, in beauty device 1, the current value can be changed in accordance with user's preference with respect to a wide application level from 100 µA to 700 µA, and electricity can be applied to the skin of the user with an appropriate current value in accordance with the user.

In addition, in the first exemplary embodiment, the current value to be applied to the skin of the user may be a value between 100 µA and 300 µA. Thus, in beauty device 1, in a case where the current value to be applied to the skin is in a range of 100 µA to 300 µA, the skin care effect is low, but stimulation is suppressed. That is, in a case where the current value to be applied to the skin is in the range of 100 µA to 300 µA, the current value is appropriate for the user suitable for weak stimulation by the application. Therefore, beauty device 1 can apply electricity to the skin of the user with an appropriate current value in accordance with the user.

In addition, in the first exemplary embodiment, the current value to be applied to the skin of the user may be a value between 300 µA and 500 µA. Thus, in a case where the current value to be applied to the skin is in a range of 300 µA to 500 µA, the current value of beauty device 1 is appropriate for the user who wants to obtain stimulation by the application and the skin care effect in a well-balanced manner. Therefore, beauty device 1 can apply electricity to the skin of the user with an appropriate current value in accordance with the user.

In addition, in the first exemplary embodiment, the current value to be applied to the skin of the user may be a value between 500 µA and 700 µA. In this case, a high skin care effect can be expected for the skin of the user. Thus, in beauty device 1, in a case where the current value to be applied to the skin is in the range of 500 µA to 700 µA, although discomfort caused by the application may be increased depending on the user, the current value is appropriate for the user who wants to obtain a high skin care effect by the application. Therefore, beauty device 1 can apply electricity to the skin of the user with an appropriate current value in accordance with the user.

### (Second exemplary embodiment)

As described above, although one specific exemplary embodiment has been described, the above-described exemplary embodiment is an example and does not limit the exemplary embodiment. For example, in the above-described exemplary embodiment, the mode in which current value switching unit 12 is a switch provided on the outer shell of beauty device 1 and the user can set the current value has been illustrated. Here, in beauty device 1 according to a second exemplary embodiment in which current value switching unit 12 is not provided as the switch on the outer shell of beauty device 1 and the current value is set inside a housing of beauty device 1, a configuration different from the configuration of beauty device 1 according to the first exemplary embodiment will be described.

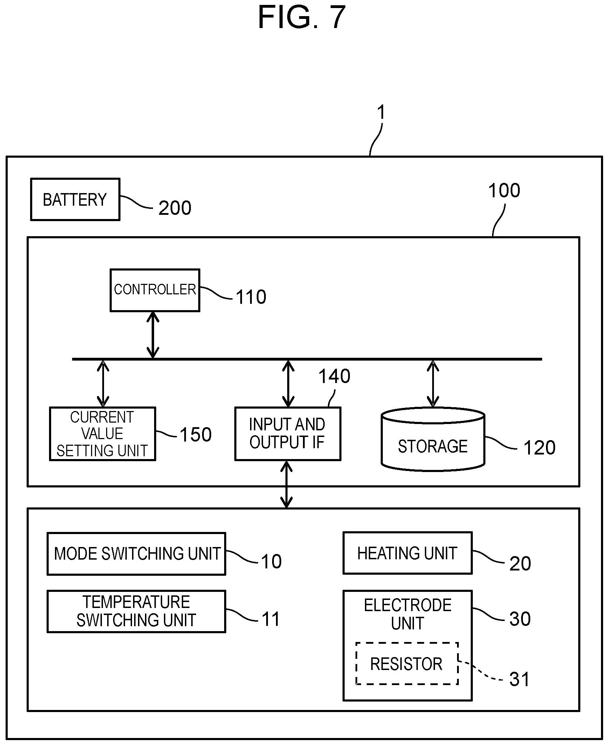

Fig. 7 is a block diagram illustrating a configuration of beauty device 1 according to the second exemplary embodiment. As illustrated in Fig. 7, beauty device 1 according to the second exemplary embodiment is different from beauty device 1 according to the first exemplary embodiment in that control board 100 includes current value setting unit 150. In the second exemplary embodiment, current value setting unit 150 corresponds to the current value switching unit.

In the second exemplary embodiment, current value setting unit 150 sets a predetermined current value at a factory at the time of shipment of beauty device 1. For example, in the second exemplary embodiment, resistor 31 of electrode unit 30 includes a plurality of resistors, and the current value is controlled by disconnecting the resistors to have a predetermined current value in accordance with the content set by current value setting unit 150.

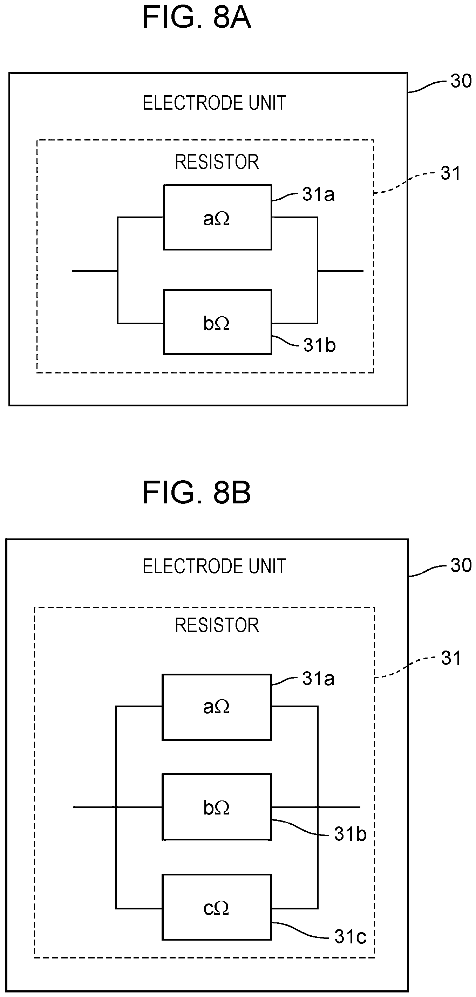

Figs. 8A and 8B are diagrams for explaining control of resistors in resistor 31. In Fig. 8A, a resistance value of resistor 31a is a S2 (ohm) and a resistance value of resistor 31b is b Ω. For example, in a case where neither resistor 31a nor resistor 31b is disconnected, when a resistance value of resistor 31 is X Ω, the resistance value of resistor 31 can be expressed by 1/X = (1/a) + (1/b). In addition, in a case where resistor 31b is disconnected, the resistance value of resistor 31 is a Ω, and in a case where resistor 31a is disconnected, the resistance value of resistor 31 is b S2.

Similarly, in addition to the configuration of Fig. 8A, in Fig. 8B, resistor 31c having a resistance value of c S2 is connected in parallel to resistor 31a and resistor 31b. Similarly to the example in Fig. 8A, in the example in Fig. 8B, for example, in a case where none of resistor 31a, resistor 31b, and resistor 31c is disconnected, when the resistance value of resistor 31 is set to X Ω, the resistance value of resistor 31 can be expressed by 1/X = (1/a) + (1/b) + (1/c). In addition, in a case where resistor 31c is disconnected, when the resistance value of resistor 31 is set to X Ω, the resistance value of resistor 31 can be expressed by 1/X = (1/a) + (1/b). Similarly, in a case where resistor 31b is disconnected, when the resistance value of resistor 31 is X Ω, the resistance value of resistor 31 1/X = (1/a) + (1/c) ohm. Similarly, in a case where resistor 31a is disconnected, when the resistance value of resistor 31 is set to X Ω, the resistance value of resistor 31 can be expressed by 1/X = (1/b) + (1/c).

Further, in the example illustrated in Fig. 8B, in a case where resistor 31b and resistor 31c are disconnected, the resistance value of resistor 31 can be expressed by a Ω. Similarly, in a case where resistor 31a and resistor 31c are disconnected, the resistance value of resistor 31 can be expressed by b Ω. In addition, in a case where resistor 31a and resistor 3 1b are disconnected, the resistance value of resistor 31 can be expressed by c Ω.

The number of resistors illustrated in Figs. 8A and 8B is not limited to two or three, and a more detailed current value can be set by increasing the number of resistors.

In the examples illustrated in Figs. 8A and 8B, although the example in which the resistor provided in electrode unit 30 is disconnected has been described, resistor 31 is not limited to be provided in electrode unit 30. For example, resistor 31 may be provided inside current value setting unit 150 of control board 100. Alternatively, resistor 31 may be realized by a configuration different from the configuration of current value setting unit 150 of control board 100.

As described above, in beauty device 1 according to the second exemplary embodiment, the example in which current value switching unit 12 for switching the current value to be applied to the skin from electrode unit 30 is not provided on the outer shell of beauty device 1 and current value setting unit 150 corresponding to current value switching unit 12 is provided inside the housing of beauty device 1 has been described. Thus, for example, a predetermined current value can be set at the time of shipment of beauty device 1, variations of products are increased, and beauty device 1 for a specific user can be realized.

### (Other exemplary embodiments)

Although the first and second exemplary embodiments have been described above, the exemplary embodiment is not limited thereto, and various modifications can be made within the scope of the gist of the exemplary embodiment. In addition, some or all of various exemplary embodiments may be combined to form a new exemplary embodiment. That is, since the above-described exemplary embodiments illustrate the technique in the present disclosure, various modifications, substitutions, additions and omissions can be performed within the scope of claims and equivalent scope of claims.

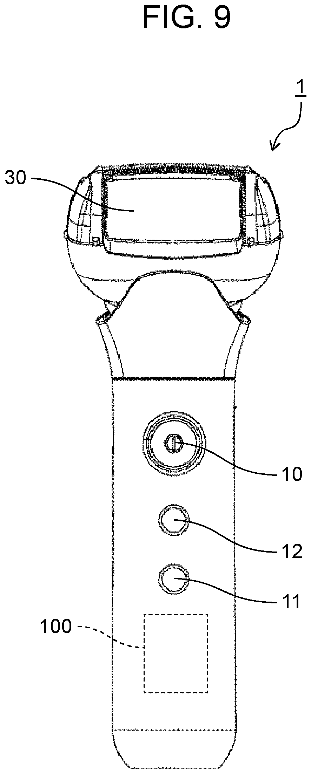

In the first and second exemplary embodiments described above, the example in which beauty device 1 is a skin care device has been described. For example, beauty device 1 may be applied to a device such as an electric razor (that is, electric razor) used by being brought into contact with the skin of a user. Fig. 9 illustrates an example in a case where beauty device 1 is applied to the electric razor. In the example illustrated in Fig. 9, mode switching unit 10, temperature switching unit 11, current value switching unit 12, electrode unit 30, and control board 100 are the same as the units illustrated in Figs. 1A and 1B. As described above, beauty device 1 is applied to the device used while being brought in contact with the skin of the user, and thus, it is possible to provide a beauty function in addition to the function of the device.

Hereinafter, features of beauty device 1 and electric razor according to the present exemplary embodiment will be described.
(1) Beauty device 1 that applies electricity to skin of a user has the following configuration.
   (i) Electrode unit 30 that is brought into contact with the skin of the user to apply electricity to the skin of the user is included.
   (ii) Current value switching unit 12 that switches a current value for applying electricity to the skin of the user from electrode unit 30 is included.
   (iii) Current value controller 116 that controls the current value to be applied from electrode unit 30 based on the current value switched by current value switching unit 12 is included.
   (iv) Current value controller 116 controls the current value such that an application level of the current value to be applied from electrode unit 30 becomes a value in a range of 100 µA to 700 µA.

In other words, beauty device 1 according to the present disclosure is beauty device 1 that applies an electricity to skin of a user. The device includes electrode unit 30 that is brought into contact with the skin of the user to apply the electricity to the skin of the user, current value switching unit 12 that switches a current value to be applied to the skin of the user from electrode unit 30, and electrical circuitry. In operation, the electrical circuitry controls the current value to be applied from electrode unit 30 based on the current value switched by current value switching unit 12, and controls the current value such that an application level of the current value to be applied from electrode unit 30 is a value in a range of 100 µA to 700 µA.

Examples of the electrical circuitry are semiconductor devices, such as an integrated circuit (commonly referred to as "IC") or a large scale integrated circuit (commonly referred to as "LSI").

According to the present disclosure, beauty device 1 can apply electricity to the skin of the user with an appropriate current value in accordance with the user by controlling the current value such that the application level of the current value to be applied from electrode unit 30 becomes a value in the range of 100 µA to 700 µA.

(2) Beauty device 1 may further have the following configuration.

(v) Storage 120 that stores the used current value may be further included.

According to this configuration, in beauty device 1, the user does not need to set an appropriate current value for each treatment, and can operate beauty device 1 with an optimal current value.

(3) Beauty device 1 may further have the following configuration.

(vi) The current value to be applied to the skin of the user may be a value between 100 µA and 300 µA.

According to this configuration, in beauty device 1, in a case where the current value to be applied to the skin is in the range of 100 µA to 300 µA, the skin care effect is low, but stimulation is suppressed. That is, in a case where the current value to be applied to the skin is in the range of 100 µA to 300 µA, the current value is appropriate for the user suitable for weak stimulation by the application. Therefore, beauty device 1 can apply electricity to the skin of the user with an appropriate current value in accordance with the user.

(4) Beauty device 1 may further have the following configuration.

(vii) The current value to be applied to the skin of the user may be a value in a range of 300 µA to 500 µA.

According to this configuration, in beauty device 1, in a case where the current value to be applied to the skin is in the range of 300 µA to 500 µA, the current value is appropriate for the user who wants to obtain stimulation by the application and the skin care effect in a well-balanced manner. Therefore, beauty device 1 can apply electricity to the skin of the user with an appropriate current value in accordance with the user.

(5) Beauty device 1 may further have the following configuration.

(viii) The current value to be applied to the skin of the user may be a value between 500 µA and 700 µA.

According to this configuration, in beauty device 1, in a case where the current applied to the skin is in the range of 500 µA to 700 µA, although discomfort caused by the application may be increased depending on the user, the current value is appropriate for the user who wants to obtain a high skin care effect by the application. Therefore, beauty device 1 can apply electricity to the skin of the user with an appropriate current value in accordance with the user.

(6) Beauty device 1 may further have the following configuration.

(ix) Current value switching unit 12 may be present on an outer shell of beauty device 1, and the current value is changeable by the user.

According to this configuration, in beauty device 1, the current value can be changed in accordance with user's preference, and electricity can be applied to the skin of the user with an appropriate current value in accordance with the user.

(7) Beauty device 1 may further have the following configuration.

(x) Current value switching unit 12 may be a switch that allows the user to freely set the application level of the current value flowing from electrode unit 30 to be in a range of 100 µA to 700 µA.

According to this configuration, in beauty device 1, the current value can be changed in accordance with user's preference with respect to the wide range of application level from 100 µA to 700 µA, and electricity can be applied to the skin of the user with an appropriate current value in accordance with the user.

(8) Beauty device 1 may further have the following configuration.

(xi) Current value switching unit 12 may be present inside a housing of beauty device 1.

According to this configuration, beauty device 1 can be set to a predetermined current value at the time of shipment of beauty device 1, variations of products can be increased, and beauty device 1 for a specific user can be realized.

(9) An electric razor may include beauty device 1 described above.

According to this configuration, the electric razor can have a beauty function in addition to an original function of the device by being applied to the device in which beauty device 1 is brought into contact with the skin of the user.

### INDUSTRIAL APPLICABILITY

The present disclosure is applicable to a beauty device that performs skin care by being brought into contact with the skin of a user to apply electricity to the skin of the user. Specifically, the present disclosure is applicable to professional and home skin care devices and electric razors.

### REFERENCE MARKS IN THE DRAWINGS

- 1: beauty device
- 10: mode switching unit
- 11: temperature switching unit
- 12: current value switching unit
- 20: heating unit
- 30: electrode unit
- 31: resistor
- 31a: resistor
- 31b: resistor
- 31c: resistor
- 100: control board
- 110: controller
- 111: mode information acquisition unit
- 112: temperature information acquisition unit
- 113: current value acquisition unit
- 114: mode controller
- 115: temperature controller
- 116: current value controller
- 120: storage
- 121: mode information DB
- 122: temperature information DB
- 123: current value information DB
- 140: input and output IF
- 150: current value setting unit
- 200: battery

## Claims

1. A beauty device that applies an electricity to skin of a user, the device comprising:
an electrode unit that is brought into contact with the skin of the user to apply the electricity to the skin of the user;
a current value switching unit that switches a current value to be applied to the skin of the user from the electrode unit; and
a current value controller that controls the current value to be applied from the electrode unit based on the current value switched by the current value switching unit,
wherein
the current value controller controls the current value such that an application level of the current value to be applied from the electrode unit is a value in a range of 100 µA to 700 µA.

2. The beauty device according to Claim 1, further comprising:
a storage that stores the current value previously used.

3. The beauty device according to Claim 1 or 2, wherein
the current value to be applied to the skin of the user is a value in a range of 100 µA to 300 µA.

4. The beauty device according to Claim 1 or 2, wherein
the current value to be applied to the skin of the user is a value in a range of 300 µA to 500 µA.

5. The beauty device according to Claim 1 or 2, wherein
the current value to be applied to the skin of the user is a value in a range of 500 µA to 700 µA.

6. The beauty device according to any one of Claims 1 to 5, wherein
the current value switching unit is present on an outer shell of the beauty device, and the current value is changeable by the user.

7. The beauty device according to Claim 6, wherein
the current value switching unit is a switch that allows the user to freely set the application level of the current value to be applied from the electrode unit to be in a range of 100 µA to 700 µA.

8. The beauty device according to any one of Claims 1 to 5, further comprising:
a housing,
wherein the current value switching unit is present inside the housing.

9. An electric razor comprising the beauty device according to any one of Claims 1 to 8.
